# EUROPEAN PATENT APPLICATION

(11) **EP 4 678 158 A1**
(43) Date of publication of application: **14.01.2026**
(21) Application number: 23800723.1
(22) Date of filing: 07.03.2023
(51) Int. Cl.: A61K 8/18, A61K 8/97, A61K 8/9789, A61K 8/9794, A61Q 19/08

(54) **ANTIOXIDANT COSMETIC COMPLEX, COSMETIC COMPOSITION, USE AND METHOD FOR COSMETIC TREATMENT**

(71) Applicant: Natura Cosméticos S.A., 05106-000 São Paulo SP (BR)
(72) Inventor: CARVALHÃES LAGO, Juliana, 05106-000 São Paulo. SP (BR); ABRAHÃO DIAS, Ana Luisa, São Paulo - SP 05106-000 (BR)
(74) Representative: Simmons & Simmons
(86) International application number: PCT/BR2023/050077
(87) International publication number: WO 2024/182863

(57) **Abstract**

The present invention deals with an antioxidant cosmetic complex that acts against damage caused by oxidative stress and free radicals on the skin and/or scalp, comprising bioactive ingredients from *Euterpe oleracea* (açaí), *Theobroma cacao* (cocoa) and *Inga edulis* (inga bean), as well as a cosmetic composition containing the same, use thereof and method.

## Description

### FIELD OF THE INVENTION

The present invention deals with an antioxidant cosmetic complex that acts against damage caused by oxidative stress and free radicals on the skin and/or scalp, comprising bioactive ingredients from *Euterpe oleracea* (açaí), *Theobroma cacao* (cocoa) and *Inga edulis* (inga bean), as well as a cosmetic composition containing the same, use thereof and method.

### BACKGROUND OF THE INVENTION

The concept of nourishing a tissue means providing an adequate metabolic balance and redox homeostasis so that this tissue can perform its perform its function in the best way possible. In terms of photoprotection, it is important to assess whether the active ingredients help maintain the redox balance of key cells for maintaining skin homeostasis, for example, keratinocytes, as well as how much these active ingredients can prevent the formation of structures that reduce the cell's ability to survive.

One of the most widely accepted theory of human aging proposes that aging is the result of cellular aging, in which an increasing number of cells reach senescence, a terminal stage in which cells cease dividing. This limits the body's or tissue's ability to regenerate itself and respond to injury or stress. This process will take place naturally over time and with the number of cell divisions, which tends to gradually decrease with each successive division, up to the point of replicative senescence.

Cellular aging can also be activated extrinsically, especially in skin that survives being exposed to the exposome. Totally avoiding sun exposure has never been an alternative for humans, since health of the skin and the individual depends on continuous stimuli of sunlight. On the other hand, exposure to the sun is the major factor causing redox imbalance, which can cause cell death or senescence, or in the worst-case scenario, induce malignant transformation. Therefore, avoiding the redox imbalance through antioxidant alternatives is key to the development of anti-aging skin products.

Cellular homeostasis is maintained by the balance between the rate at which Reactive Oxygen Species (ROS) and Reactive Nitrogen Species (RNS) are formed and the rate of suppression of these species, which balance is finely controlled by redox signaling mechanisms. However, the rate at which oxidizing species are produced can greatly exceed the capacity of the maintenance systems and an imbalance situation is reached. Redox imbalance is involved in a series of physiological conditions and can lead to the formation of advanced glycation or lipid peroxidation products, which hinder cell survival and facilitate cellular aging.

Identification of free radical reactions as promoters of the aging process and the appearance of the first signs of aging, including premature graying of hair, implies interventions aimed at limiting or inhibiting them.

Plant bioactives have been researched, since in addition to their direct antioxidant effect, they can also exert an antioxidant protective effect through the protection of membranes. The relationship between the antioxidant capacity and efficiency of membrane protection can be used to measure the performance of plant bioactives. The ability of a given compound to protect biological membranes is related not only to the antioxidant capacity of the bioactive, but also to the level of interaction with the biological membrane.

Thus, there remains the search in the art for new bioactive agents or bioactive complexes capable of acting efficiently against damage caused by oxidative stress and free radicals in skin and/or scalp.

### BRIEF DESCRIPTION OF THE FIGURES

Figures 1 to 3. Results of the percentage of antioxidant protection for the tested samples.
Figure 4. Absorption transient of triplet species (derived from methylene blue) after laser photon excitation at 660nm in the absence and in the presence of increasing concentrations of beta-carotene. All samples were purged with argon for 5 minutes to reduce the dissolved oxygen concentration.
Figure 5. Lifespan of the triplet as a function of beta-carotene concentration.
Figure 6. Suppression constant calculated using the Stern-Volver model and using only the lifespans at the 4 lowest concentrations of beta-carotene.

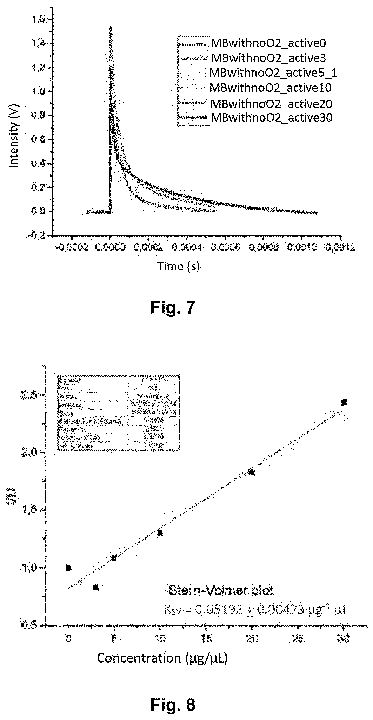

Figure 7. Absorption transient at 470nm of triplet species (derived from methylene blue) after laser photon excitation at 660nm in the absence and in the presence of increasing concentrations of active 6714. All samples were purged with argon for 5 minutes to reduce the dissolved oxygen concentration.
Figure 8. Suppression constant calculated using the Stern-Volver model in the presence of increasing concentrations of active 6714.

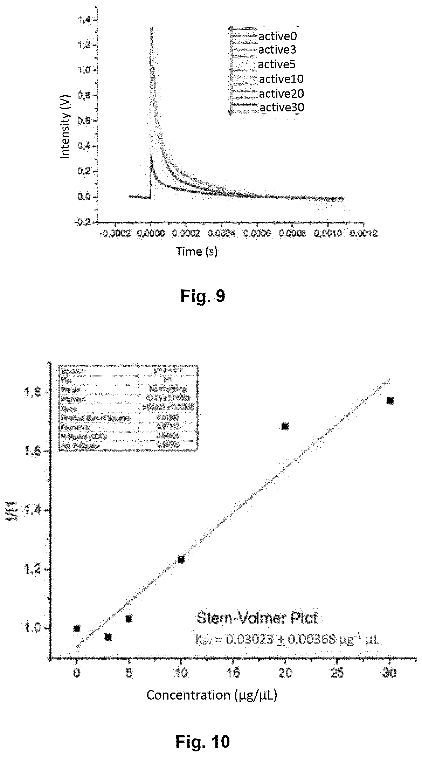

Figure 9. Absorption transient at 470nm of triplet species (derived from methylene blue) after laser photon excitation at 660nm in the absence and in the presence of increasing concentrations of active 55107. All samples were purged with argon for 5 minutes to reduce the dissolved oxygen concentration.
Figure 10. Suppression constant calculated using the Stern-Volver model in the presence of increasing concentrations of active 55107.

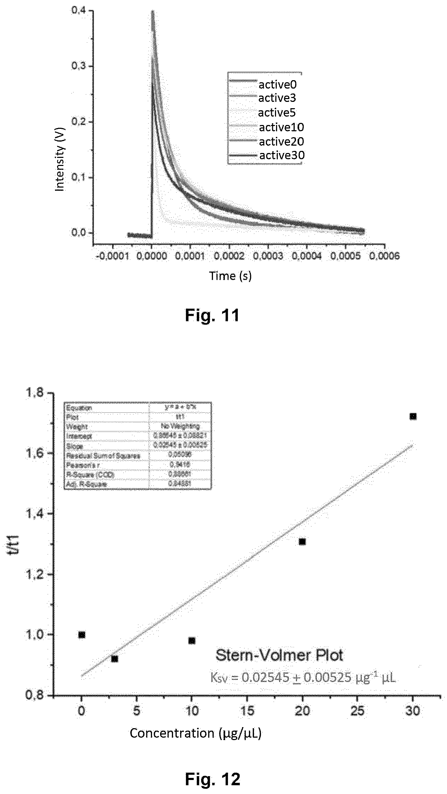

Figure 11. Absorption transient at 470nm of triplet species (derived from methylene blue) after laser photon excitation at 660nm in the absence and in the presence of increasing concentrations of active 55825. All samples were purged with argon for 5 minutes to reduce the dissolved oxygen concentration.
Figure 12. Suppression constant calculated using the Stern-Volver model in the presence of increasing concentrations of active 55825.
Figure 13. Absorption transient at 400nm of triplet species (derived from methylene blue) after laser photon excitation at 660nm in the absence and in the presence of increasing concentrations of active 9617. All samples were purged with argon for 5 minutes to reduce the dissolved oxygen concentration.
Figure 14. Suppression constant calculated using the Stern-Volver model in the presence of increasing concentrations of active 9617.

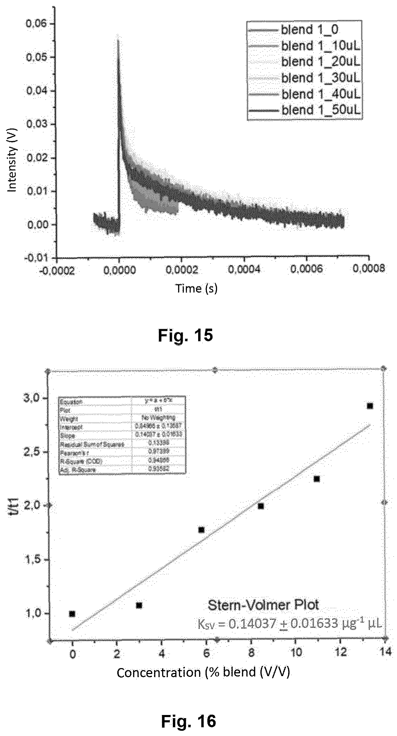

Figure 15. Absorption transient at 470nm of triplet species (derived from methylene blue) after laser photon excitation at 660nm in the absence and in the presence of increasing concentrations of blend 1. All samples were purged with argon for 5 minutes to reduce the dissolved oxygen concentration.
Figure 16. Suppression constant calculated using the Stern-Volver model in the presence of increasing concentrations of blend 1. The suppression constant was expressed in % (vol:vol).

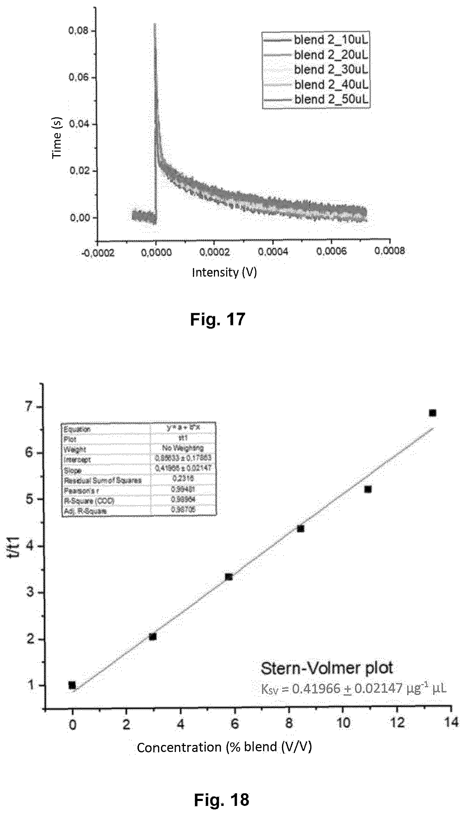

Figure 17. Absorption transient at 470nm of triplet species (derived from methylene blue) after laser photon excitation at 660nm in the absence and in the presence of increasing concentrations of blend 2. All samples were purged with argon for 5 minutes to reduce the dissolved oxygen concentration.
Figure 18. Suppression constant calculated using the Stern-Volver model in the presence of increasing concentrations of blend 2. The suppression constant was expressed in % (vol:vol).

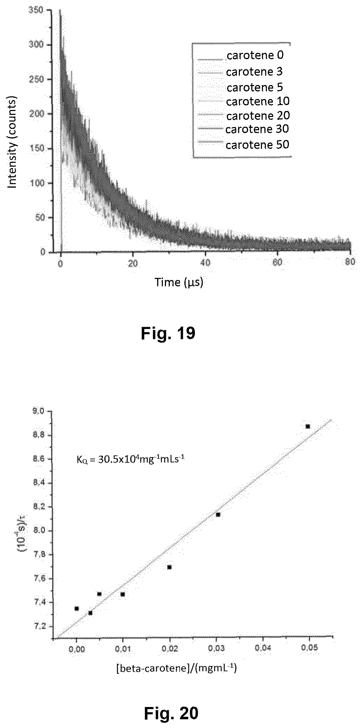

Figure 19. Transient of phosphorescence emission at 1270nm in ethanol in the absence and in the presence of increasing concentrations of beta-carotene.
Figure 20. 1O2 suppression constant calculated using the Stern-Volver model in the presence of increasing concentrations of beta-carotene.

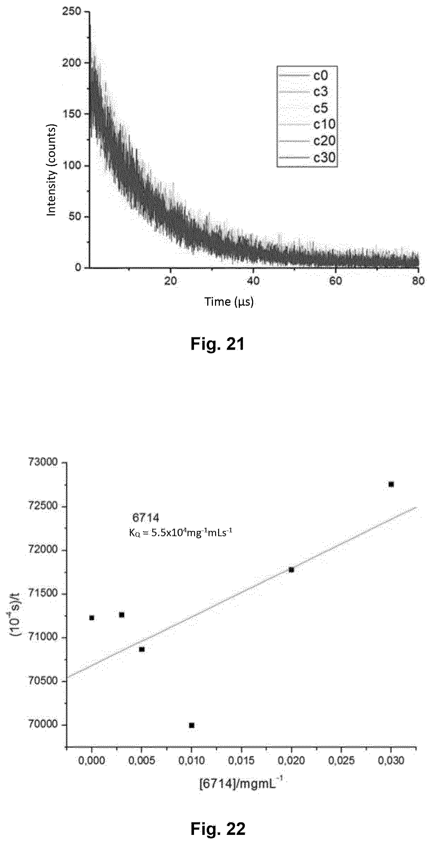

Figure 21. Transient of phosphorescence emission at 1270nm in ethanol in the absence and in the presence of increasing concentrations of active 6714.
Figure 22. 1O2 suppression constant calculated using the Stern-Volver model in the presence of increasing concentrations of active 6714.

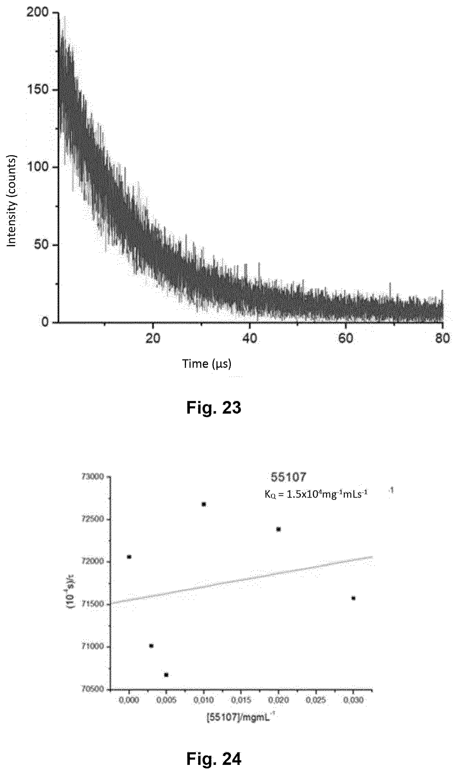

Figure 23. Transient of phosphorescence emission at 1270nm in ethanol in the absence and in the presence of increasing concentrations of active 55107.
Figure 24. 102 suppression constant calculated using the Stern-Volver model in the presence of increasing concentrations of active 55107.

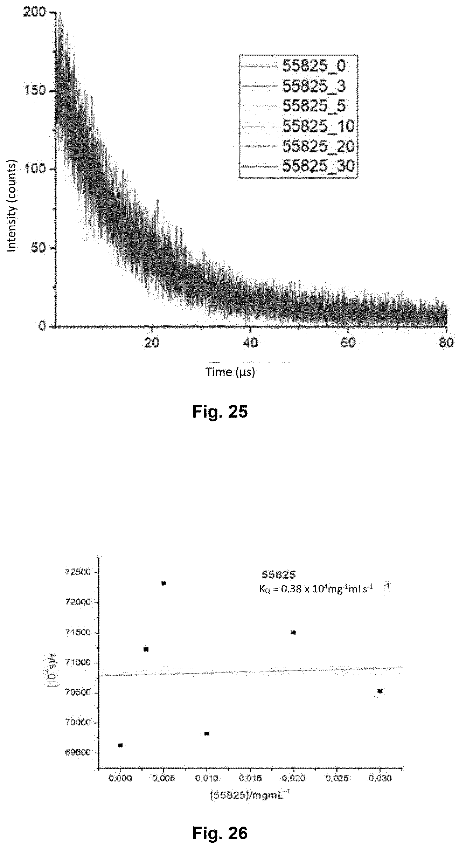

Figure 25. Transient of phosphorescence emission at 1270nm in ethanol in the absence and in the presence of increasing concentrations of active 55825.
Figure 26. 102 suppression constant calculated using the Stern-Volver model in the presence of increasing concentrations of active 55825.
Figure 27. % CF release as a function of the time of irradiation using soy lecithin liposomes, 15 µM photosensitizer (DMMB) and different samples in 10 mM Tris buffer, 0.3 M sodium chloride (pH = 8). (A and B) plate in the dark and (C and D) irradiated plate.
Figure 28. Viability expressed as the percentage of MTT reduction by HaCat cells with no irradiation and 24 hours after UVA irradiation (12 J cm⁻²). *EtOH1 and EtOH2 controls correspond to the amount of ethanol in Blend1 and Blend2, respectively. Blend 1 - 6714 (0.01 mg/mL) + 55107 (0.02 mg/mL) + 55285 (0.02 mg/mL); Blend 2 - 6714 (0.01 mg/mL) + 9617 (0.01 mg/mL) + α-tocopherol (0.01 mg/mL).
Figure 29. Accumulation of lipofuscin detected by Sudan Black B staining 48 hours after UVA irradiation (12 J cm⁻²) of HaCaT cells.
Figure 30. Quantification of particles stained with Sudan Black B in HaCaT cells, 48 hours after UVA irradiation (12 J cm⁻²).

### DESCRIPTION OF THE INVENTION

The present invention deals with an antioxidant cosmetic complex that acts against damage caused by oxidative stress and free radicals on the skin and/or scalp, comprising bioactive ingredients from *Euterpe oleracea* (açaí palm), *Theobroma cacao* (cocoa) and *Inga edulis* (inga bean).

The bioactive ingredients according to the present invention can be obtained by processes known in the art, from specific parts of plant species. Without imposing any limitation, for example, they can be obtained from aerial plant parts, such as stalks, leaves and flowers, or just leaves as well as from agro-industrial residues such as seeds, mesocarp fiber or mucilage.

In a particular embodiment, the present invention deals with an antioxidant cosmetic complex that acts against damage caused by oxidative stress and free radicals in the skin and/or scalp, which comprises, relative to the total weight of the cosmetic composition:
(a) from about 0.0002% to about 20% of *Euterpe oleracea;*
(b) from about 0.0001% to about 20% of *Theobroma cacao;*
(c) from about 0.0002% to about 20% of *Inga edulis.*

The above percentages as well as those indicated in the following examples are based on the total weight of the final cosmetic composition in which the complex will be delivered.

In another embodiment, the present invention further contemplates, comprehensively, cosmetic compositions comprising the antioxidant cosmetic complex according to the present invention, together with cosmetically acceptable excipients.

The cosmetically acceptable excipients according to the present invention are those known to the person skilled in the art for making cosmetic bases in various forms, for example, emulsions, creams, gels, serums, and others known to the person skilled in the art. For example, without any limitation, cosmetically acceptable excipients can be selected from those cited in the "International Cosmetic Ingredient Dictionary & Handbook", 16th Edition.

In another embodiment, the present invention further deals with the use of the antioxidant cosmetic complex to manufacture a cosmetic composition to act against damage caused by oxidative stress and free radicals in the skin and/or scalp.

Also, in another embodiment, the present invention contemplates a method for topical cosmetic treatment of damage caused by oxidative stress and free radicals in the skin and/or scalp, which comprises applying to the skin and/or scalp, whether in need thereof or not, an effective amount of the complex or the cosmetic composition according to the present invention. Therefore, the method discussed herein can be a prevention or cosmetic treatment.

The following examples, without imposing any limitation, show the particular embodiments of the present invention as well as demonstrating the effectiveness of the complex according to the present invention in treating damage caused by oxidative stress and free radicals.

### EXAMPLES

Different bioactives were comparatively assessed for their antioxidant potential and potential synergy when evaluated together, in order to develop new ingredients to act efficiently in cosmetic compositions against the damage caused by oxidative stress in the skin and/or scalp.

In this sense, the antioxidant potential of six plant ingredients was assessed through the neutralization of the DPPH molecule in a proposed biochemical evaluation and in a cell culture of primary fibroblasts.

Furthermore, the suppression of excited states (triplets and singlet oxygen) was also measured by comparatively assessing the ability to suppress the active states and the membrane protection was evaluated (spectroscopic tests with liposomes). The results were compared with gallic tannin (the most efficient membrane protector known to date).

The resulting anti-aging activity of the tested ingredients was assessed in human keratinocytes (HaCaT). Viability protection and anti-aging activity were tested in a UVA-induced damage model. The endpoint was the level of accumulated liposfucin with and without the treatments. Cell viability was monitored by MTT and the anti-aging ability was assessed by a decrease in lipofuscin accumulation.

The analyzed samples were identified as:
55181: Brazilian peppertree (*aroeira*) extract - solubilization in ethanol
53797: wild sage (*guagatonga*) extract - solubilization in water
57713: paracress (*jambu*) extract - solubilization in water
6714: cocoa extract - solubilization in ethanol
55107: inga bean extract - solubilization in water
55285: açaí palm bioactive - solubilization in water
9617: green tea - solubilization in water
α-tocopherol

Samples 9617 and α-tocopherol were only considered in the Blend 1 x Blend 2 comparison.

In the examples that follow, "blend" means tested mixtures or complexes containing more than one bioactive ingredient.

Concentrations for excited state suppression, membrane protection and cell viability (MTT) experiments were:
6714: 0.01 mg/mL)
55107: 0.02 mg/mL)
55285: 0.02 mg/mL
Blend 1 - 6714 (0.01 mg/mL) + 55107 (0.02 mg/mL) + 55285 (0.02 mg/mL)
Blend 2 - 6714 (0.01 mg/mL) + 9617 (0.01 mg/mL) + α-Tocopherol (0,01 mg/mL)

Concentrations for anti-aging experiments (lipofuscin accumulation):
Blend 1 - 6714 (0.01 mg/mL) + 55107 (0.02 mg/mL) + 55285 (0.02 mg/mL)
Blend 2 - 6714 (0.01 mg/mL) + 9617 (0.01 mg/mL) + α-Tocopherol (0,01 mg/mL)

### Example 1 - Biochemical DPPH

The biochemical test using DPPH (2,2-diphenyl-1-picryl-hydrazyl) is based on the reduction of a stable free radical in an ethanol solution by substances having antioxidant activity. DPPH reduction is observed by the decrease in absorbance, since in its radical form DPPH has a violet color that absorbs at 517nm. In the presence of an antioxidant or other radical species, absorption disappears or decreases. Therefore, the use of DPPH makes it possible to evaluate the antioxidant potential of different chemical compounds, as well as those present in plant extracts.

The results are obtained through the IC50, which shows the minimum concentration required to neutralize 50% of DPPH free radicals in solution.

**Table 1 IC₅₀ of the samples tested alone.**

| Sample | IC₅₀ (mg/mL) |
|---|---|
| Açaí palm - bioactive | 0.02 |
| Brazilian peppertree | 0.05 |
| Cocoa | 0.01 |
| wild sage | 0.09 |
| Inga bean | 0.02 |
| paracress | 0.7 |

From these values, all samples were tested again using the IC₅₀ concentration with the samples combined in groups of three to evaluate the synergy of protection with combined use.

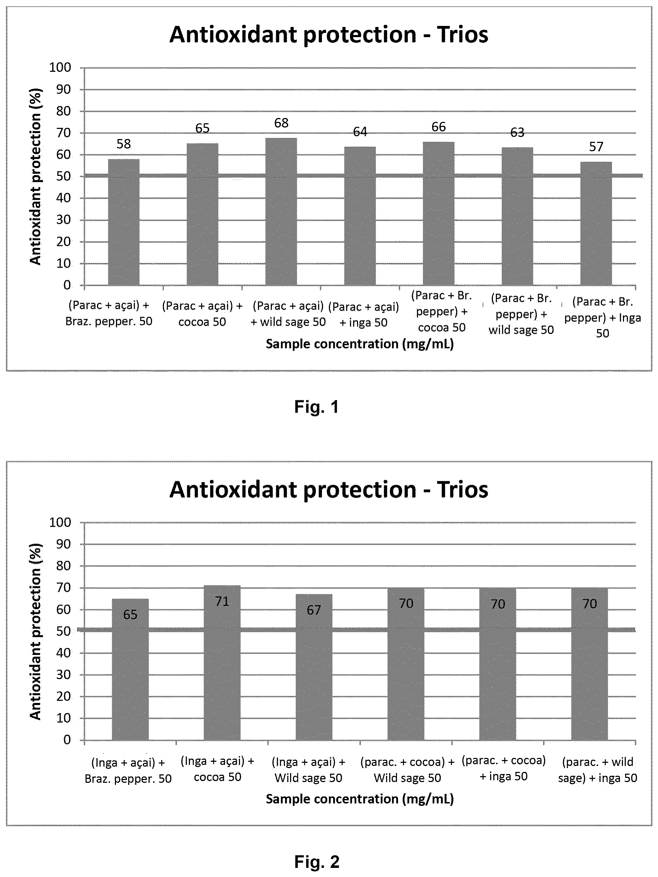

It is possible to observe in Figures 1 to 3 that all combinations surpassed the 50% protection achieved with the isolated samples at the IC₅₀ concentration, increasing the antioxidant protection against free radicals by up to 20% with the blend of active ingredients as compared to each active ingredient alone.

### Example 2 - Cellular DPPH

DPPH reduction tests using endogenous antioxidants are performed on human skin fibroblasts. The basis for this test, an adaptation of the traditional DPPH test, is to assess the potential production of endogenous antioxidants (produced by cells) after stimulating the cells with compounds tested at different concentrations. After exposure of the cells to the active ingredients, a DPPH-containing solution is added to the plate. Therefore, the reduction in DPPH is the result of the synthesis of endogenous antioxidants by the cells under the influence of the active.

The top five results from the combination of active ingredients in biochemical DPPH, in addition to the ingredients alone, were selected to proceed to cellular DPPH tests.

**Table 2.1 Protection percentage**

| Sample | Protection (%) |
|---|---|
| Açaí palm - bioactive | 21 |
| Brazilian peppertree | -33 |
| Cocoa | 26 |
| wild sage | 21 |
| Inga bean | 11 |
| paracress | -3 |

**Table 2.2 Protection percentage**

| Sample - *Blends* | Protection (%) |
|---|---|
| Inga bean + cocoa + wild sage | 59 |
| açaí palm + cocoa + wild sage | 61 |
| Açaí palm + cocoa + Inga bean | 62 |
| paracress + cocoa + wild sage | 25 |
| wild sage + Brazilian peppertree + cocoa | 28 |

All combinations were found to outperform the protection achieved with the isolated samples at the IC₅₀ concentration. Considering the best results from the combination of ingredients, the combination of açaí palm + cocoa + inga bean was the most efficient one in increasing the antioxidant protection against free radicals as compared to each of the active ingredients alone.

### Example 3 - Suppression of excited states

The excited state suppression ability was assessed by measuring the transient absorption of triplet states using flash photolysis and by singlet oxygen emission in the near infrared.

Singlet oxygen suppression: The detector (PMT Hamamatsu R5509 - model PC176TSCE005) was cooled to -80°C and set to 1500 V and the signals were acquired using a proprietary software from SHB Analytics GmbH (Germany). Excitation was performed by a Quantel Brilliant laser (2w-3w-Rainbow). Emission from the sample is filtered so that irradiation in the range of from 1270 ± 20nm is detected. A microcomputer is connected to the system and analysis of the transients is carried out using an ultra-fast acquisition plate. It is important to remember that luminescence emission at 1270nm is characteristic of singlet oxygen, being the safest method of detecting the presence and reactivity of this species. The decay kinetics provides direct information about the suppression efficiency of the active ingredients against singlet oxygen.

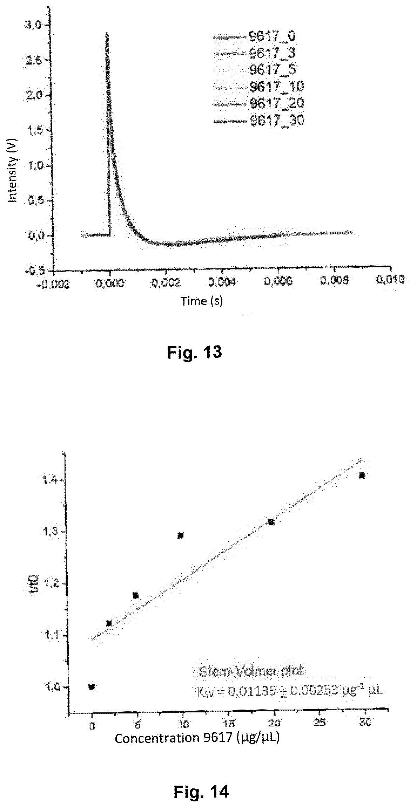

Triplet suppression: The SHB Laser Flash Photolysis system was used (Figure 14). A laser pulse (10mJ/pulse, 10-20ns) impinges on the sample providing electronic excitation. To assess the formation and the lifetime of excited states or other reactive intermediates, the change in absorbance prior to and after electronic excitation of the sample is monitored by an optical beam (LED) as a function of time. It monitors the disappearance of the species in the fundamental state or the appearance of the excited or radical species. The laser beam is positioned at 90° to the monitoring beam. Shutters are used in both the excitation and monitoring beams to protect the sample from photolysis. The detector in this technique consists of a combination of filters and diode detector. The diode response is read on a digital oscilloscope (Tektronix 2230), which data is subsequently transferred to the microcomputer. The entire equipment is controlled by a microcomputer and the time resolution of the instrument is of about 50ns. A Quantel Nd:YAG laser (2w, 3w Rainbow, pulsed at 3 ns, 80.1% power) was used. Selection of the excitation wavelength was made in the OPOTEK software at 660nm. Absorption of transients after the laser pulse was carried out in a simple photometer based on LEDs (blue, green and red) and filters. Readings were taken in a 2mm x 10mm quartz cuvette. Methylene blue (photosensitizer) samples were dissolved in the designated solvent and bubbled with argon to remove oxygen.

### Suppression of triplet species

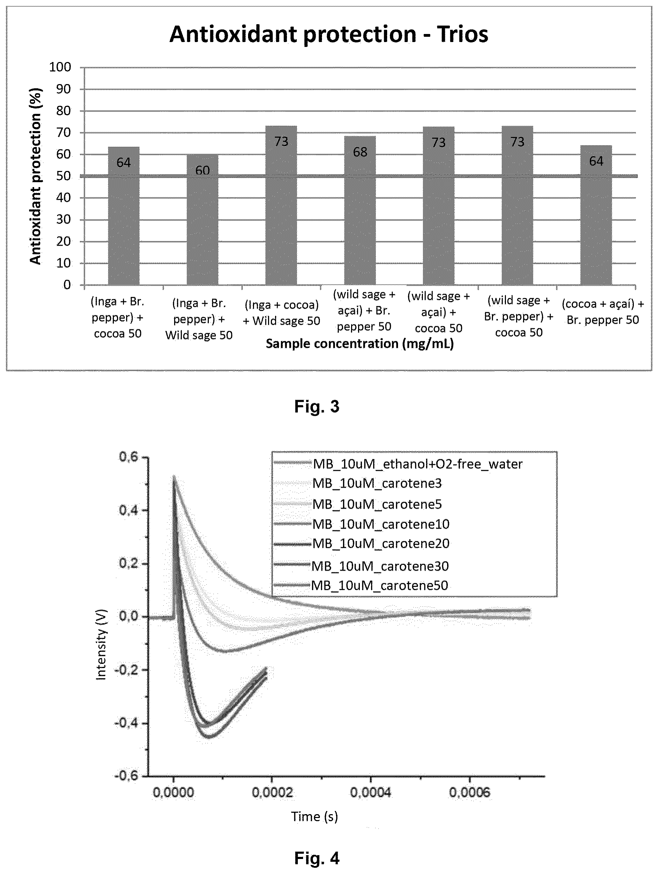

Measurements were carried out on the Laser Flash Photolysis system from SHB Analytics GmbH. A Quantel Nd:YAG laser (2w, 3w, Rainbow, pulsed at 3 ns) was used to excite the sample. The triplet was generated by exciting a 10µM solution of methylene blue in a solution purged with argon to remove oxygen. The reason we remove oxygen is to increase the triplet's lifespan and allow us to evaluate its reaction with other compounds. In the presence of oxygen (in a saturated solution), the triplet reacts with oxygen fast, preventing the assessment of suppression by other active ingredients. Formation of the triplet is observed by the transient absorption of this species, that is, by an increase and subsequent decrease in absorption at a wavelength of 470nm (Figure 4).

Note in figure 4 the orange line obtained with no addition of suppressor. The increased absorption is almost instantaneous (the ICS process takes place on a sub-nanosecond scale) and absorption of this species decays over time in the range of hundreds of microseconds due to the presence of remaining concentrations of oxygen in the solution (~500 times lower than in an air-balanced solution). In the presence of air-balanced solutions (saturated oxygen concentration) the triplet decay would take place in the sub-microsecond range. Also note in figure 4 that additions of increasing concentrations of beta-carotene cause the lifespan of the triplet species to decrease, that is, the triplet lasts less time as it is suppressed by beta-carotene. It is worth mentioning that beta-carotene was used here as a standard suppressing agent, as it efficiently suppresses both triplet species and singlet oxygen.

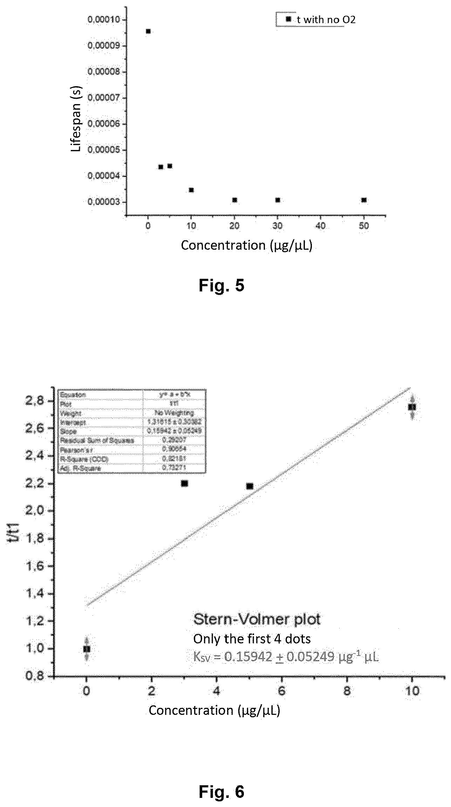

The lifespan of the triplet species is estimated by fitting the decay by exponential decay functions. Note in figure 5 that the triplet's lifespan decreases as the beta-carotene concentration increases. This decay is only linear at low concentrations of the suppressor, so that we only use the dots to estimate the suppression constant. (Figure 6). Calculation of the suppression constant was carried out using the Stern-Volmer model, in which the decay time in the presence of the active is divided by the time in its absence and this ratio (τ/τ0) is plotted as a function of the suppressor concentration (Figure 6).

All actives tested showed some level of triplet suppression, following the following order of decreasing efficiency: Beta-carotene > 6714 > 55107 > 55825 > 9617 (table 1). Both blends showed high triplet suppression efficiency, providing very clear and statistically robust Stern-Volmer plots. The individual suppression constants and the suppression constants in the blends are not comparable, as they are expressed in distant units.

**Table 3. Triplet suppression constants for actives and blends**

| Actives | Kst |
|---|---|
| Beta-carotene | 0.16 ± 0.05 (µg⁻¹µL) |
| 6714 | 0.052 ± 0.005 (µg⁻¹µ^{L}) |
| 55107 | 0.030 ± 0.004 (µg⁻¹p^{L}) |
| 55825 | 0.025± 0.005 (µg⁻¹µL) |
| 9617 | 0.011± 0.002 (µg-1µL) |
| Blend 1 | 0.14 ± 0.02 (%, vol:vol) |
| Blend 2 | 0.42 ± 0.02 (%, vol:vol) |

### Singlet oxygen suppression

To assess the actives' ability of suppressing singlet oxygen (102), the near-infrared emission (1270nm) was measured, which is characteristic of 102 and a golden standard for this detection. Beta-carotene was also used as a positive suppression standard.

The detector (PMT Hamamatsu - model H10330-45) was cooled to -80°C and set to -800 V and the signals were acquired using a proprietary software from SHB Analytics GmbH (Germany). Excitation was performed by a Quantel Brilliant Nd:YAG laser (2w-3w-Rainbow). Sample emission is detected in the range of 1270 ± 20nm. A microcomputer is connected to the system and analysis of the transients is carried out using an ultrafast acquisition plate. It is important to remember that the luminescence emission at 1270nm is characteristic of singlet oxygen, being the safest method of detecting the presence and reactivity of this species. The decay kinetics provides direct information about the suppression efficiency of the active ingredients against singlet oxygen.

In addition to beta-carotene, singlet oxygen suppression was unconditionally observed for compound 6714. Suppression constant for this active is about one order of magnitude lower than the beta-carotene standard (Table 4). Actives 55107 and 55825 were not efficient in suppressing 102. Suppression constants were calculated for illustrative purposes, but there was no correlation between suppression and the concentration of the actives. Note that the linear fit is poor with a very low R2.

**Table 4. Singlet oxygen suppression constants**

| Actives | K_{SV} (mg⁻¹ mLs⁻¹) |
|---|---|
| Beta-carotene | 30.5 x 10⁴ |
| 6714 | 5.5 x 10⁴ |
| 55107 | 1.5 x 10⁴ |
| 55825 | 0.4 x 10⁴ |

### Example 4 - Membrane protection

Soy lecithin liposomes were used as a membrane mimetic model. Oxidative damage and protection of membrane integrity were quantified using methods known in the art.

### Measurement of photoinduced carboxyfluorescein (CF) release from liposomes

Liposome suspensions were prepared using thin films of 30 mg soy lecithin and 1 mL of 50 mM CF, which were hydrated in Tris buffer (pH=8), producing a liposome suspension with CF encapsulated into the internal compartment. Non-encapsulated CF was removed by exclusion chromatography on a Sephadex G-50 column in equilibrium with 0.3 M sodium chloride in 10 mM Tris buffer (pH = 8). The liposome-containing fraction was identified visually and collected in a clean vial. Membrane damage quantification was performed in a 96-well fluorescence microplate. Volume of the liposome suspension was always 7 µL and concentration of photosensitizer 1,9 Dimethyl Methylene Blue (DMMB) was set at 15 µM (except for the control which had no photosensitizer added). Each well was then filled with 0.3 M sodium chloride in 10 mM Tris buffer (pH = 8), DMMB, and the sample solutions so that the final volume was always 300 µL. The entire microplate was irradiated with LED light at a maximum emission wavelength of 650 nm and irradiance of 13.5 mW cm⁻² (Ethik Technology^{©}, São Paulo, Brazil). Fluorescence at 517 nm (ICF) was monitored as a function of the irradiation time with a microplate reader operating at 480 nm-excitation. At the end of the experiment, 10% (v/v) Triton X-100 was added to each well and the fluorescence intensity was recorded again (ICFT). For each ICF value, the percentage of CF released (% CF released) was calculated.

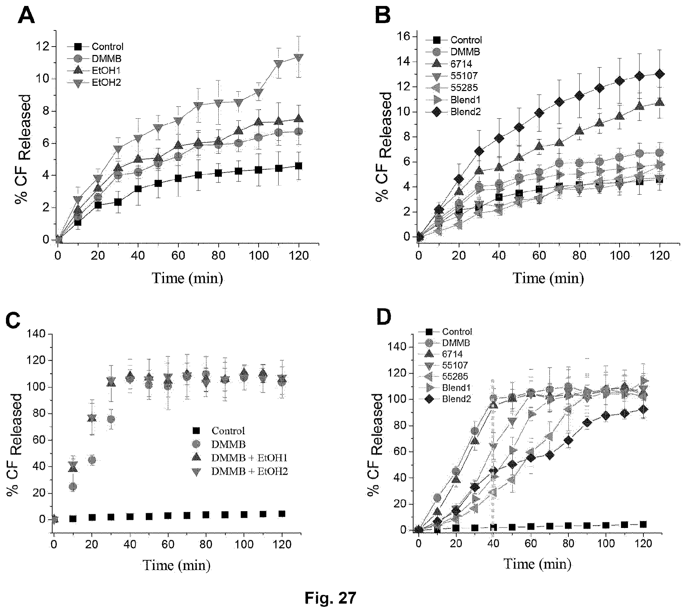

Carboxyfluorescein release was correlated with the irradiation duration (Figure 27). It should be noted that controls in the dark (Figures 27A and 27B) show a small level of CF release (about at most 12%), while liposomes irradiated with red light (Figure 27C) show a significant increase in CF release soon after the first minutes of irradiation, reaching 100% leakage within 30 minutes. This is due to the generation of triplets and singlet oxygen in the membrane, leading to the formation of several lipid peroxidation products. Especially relevant to membrane leakage is the formation of truncated lipid aldehydes, which are responsible for membrane leakage.

The purpose of the experiment is to quantify liposome leakage in the presence of Natura's active ingredients. Note that almost all actives tested, with the exception of active 6714, offer measurable levels of protection. For individual actives, the level of protection decreases in the following order: 55285>55107>6714. Of the blends, note that after 40 minutes of irradiation, Blend 1 and Blend 2 protect ~50% of the vesicles (Figure 27D).

### Termination of the method of membrane protection

The ability of the extracts to protect membranes against oxidative leakage depends on both the antioxidant capacity of the active ingredient and its ability to interact with membrane lipids.

As it is the skin's first line of protection, the corneal extract (CE) experiences several changes after exposure to the sun. CE lipid oxidation has been studied by molecular dynamics simulations. The behavior of CE oxidized lipids in a bilayer is similar to that observed for lipids oxidized in less complex model systems such as vesicles, exhibiting a significant increase in the permeability of reactive oxygen species and hence creating a self-fed oxidation cycle.

Protecting skin lipids from photoinduced oxidation is an interesting method to prevent the dissemination of damage from light exposure. However, changes in the CE that are linked to increased TWEL (*transepidermal water loss*) 48 hours after sun exposure, appear to be correlated with the hyperproliferation of keratinocytes, which no longer differentiate properly, resulting in abnormal lamellar structures in the CE. Therefore, it is also important to assess the effects of the active ingredients on keratinocytes in order to make a comprehensive hypothesis of how they will act in maintaining skin homeostasis.

### Example 5 - Measurement of anti-aging activity in human keratinocytes

Two experiments were carried out in this module. The first consisted of assessing the effect of extracts on the viability of HaCaT keratinocytes using the MTT method both in the dark (cytotoxicity) and after UVA irradiation (photoprotection). In this experiment, the effects of pure extracts 6714, 55107, 55285, as well as mixtures entitled Blend 1 and Blend 2, were evaluated. In a second experiment, we assessed the ability of blends 1 and 2 to promote anti-aging actions in HaCaT cells subjected to UVA using the accumulation of lipofuscin as the cell aging end-point.

### Viability of HaCaT cells by MTT assay

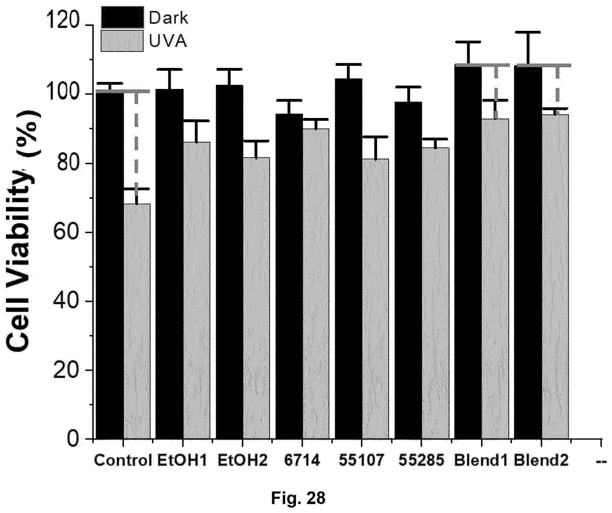

Human skin immortalized keratinocyte (HaCaT) cells were seeded (2x10⁴ cells/well) in 48-well cell culture plates. The next day, the cells were washed with PBS (2x) and incubated for 1 hour in serum-free DMEM medium containing the different samples studied. Since samples 6714 and α-tocopherol (blend 2) were prepared in ethanol, a series of controls was also analyzed with the corresponding amounts of ethanol used in the studies with these samples. After 1 hour of treatment, the cells were washed and irradiated in PBS with UVA (12 J cm⁻²). 24h after irradiation, the colorimetric assay based on MTT reduction was performed (Figure 28). HaCaT cells were incubated with a solution of 50 µg/mL MTT in 1% DMEM medium for 2 hours in a culture oven at 37°C and then solubilizing formazan with 300 µL/well of dimethyl sulfoxide (DMSO). Absorbances were measured at 550 nm using the Spectramax plate reader.

In the results presented in Figure 28, it was noted that the black bars are all similar, with viability values close to 100% for both controls and bioactives, demonstrating that none of the bioactives present measurable levels of toxicity in the dark, that is, none of the actives was shown to be cytotoxic to HaCaT cells. It is also noted that in the control (without any treatment with active ingredients or alcohol, Figure 28, bar on the left), UVA irradiation causes a decrease in keratinocyte viability from 100 to 67%. It is also noted that keratinocytes previously treated with active ingredients and even with ethanol, show an increased viability as compared to that of keratinocytes irradiated with UVA (around 20%). Keratinocytes previously treated with both blend 1 and blend 2 and irradiated show viability of ~90%, which represents a 25% increase in protection against the effects of UVA (Figure 28). It should also be noted that there is no significant difference between the photoprotection effects on keratinocyte viability of both blends, however both have greater protection capacity as compared to the untreated control (Figure 28).

### Example 5 - Anti-aging through the detection of Lipofuscin

The procedures followed the state-of-the-art practice. UVA can be deemed an agent that increases the speed of cellular aging, which occurs naturally after dozens of mitotic divisions, for an aging that is measurable within 48 hours. Oxidative damage caused by UVA in both mitochondria and lysosomes causes disruption of the autophagic fluxes in the homeostatic axes between these two organelles, which culminate in lipofuscin building up.

The immortalized human keratinocyte (HaCaT) cell line (passages 11 and 12) was grown in Dulbecco's Modified Eagle Medium (DMEM), supplemented with 10% (v/v) fetal bovine serum, 4 mM L-glutamine, 100 U/mL penicillin and 100 pg/mL streptomycin and incubated at 37 °C and 5% CO₂. HaCaT cells (2x10⁵ cells/well) were seeded on a coverslip in 6-well plates. After 24 hours, cells were washed in PBS and treated with blends 1 and 2 for 1 hour. The cells were washed again and irradiated with UVA at a dose of 12J.cm⁻² in PBS. After irradiation, the cells were washed and incubated with the medium for 48 hours. After 48 hours, the cells were washed twice with PBS, fixed with 4% (w/v) formaldehyde for 15 minutes, washed again with PBS. Then, the cells were stained with Sudan Black B solution (SBB, 0.7% in 70% ethanol) for 30 minutes, washed in distilled water and the coverslips were visualized using a Zeiss Axiovert 200 inverted microscope provided with Plan-APOCHROMAT cells. Images were obtained using bright-field transmitted light microscopy. The images were quantified using the Image-J software (NIH, Bethesda, MA, USA) by converting the TIF images into 8-bit grayscale and after adjusting the light/dark levels. The area marked with SBB was delimited by drawing the region of a cell with polygonal selection, obtaining data on the area, mean gray value and integrated image density. Values in the graphs were obtained by dividing the integrated intensity by the area, calculating the mean ± standard deviation.

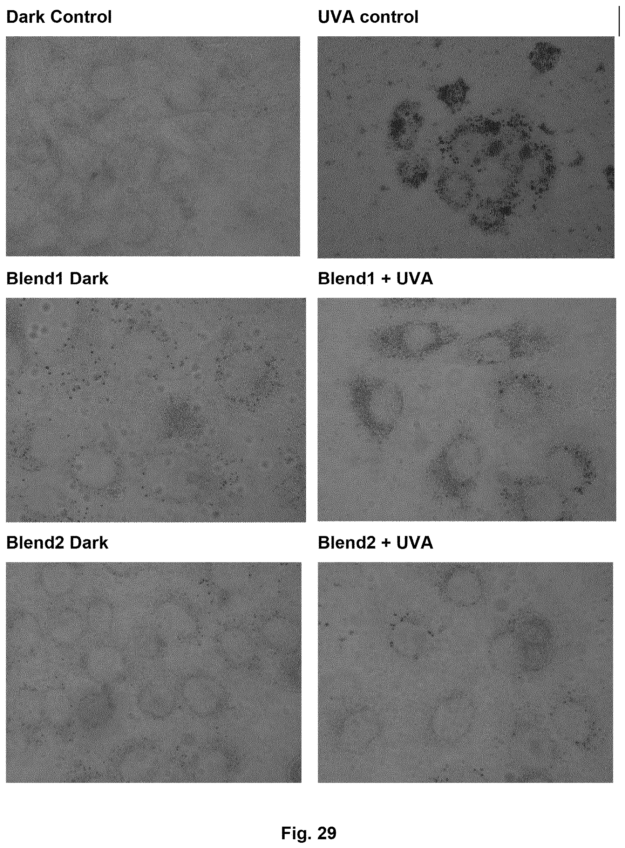

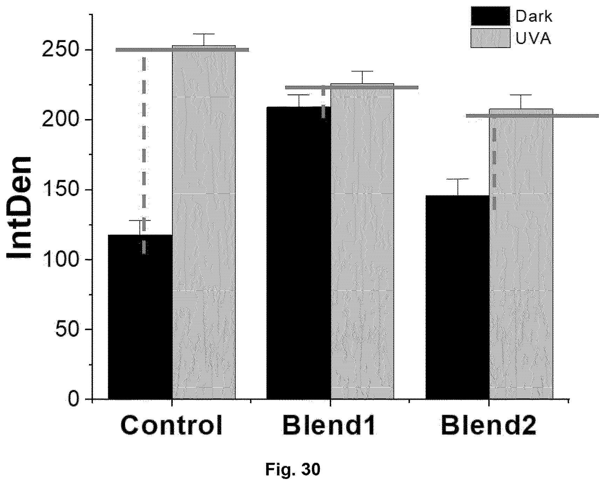

Note that control cells kept in the dark show a level of SBB staining much lower than the level of staining after UVA irradiation (Figure 29). As discussed above, this level of SBB contrast is due to the lipofuscin build up (the level of SBB staining of irradiated cells is practically twice the value in irradiated cells). Also note that blends 1 and 2 show lower contrast levels than the control, demonstrating that both blends are capable of reducing UVA-induced aging (Figures 29 and 30). When comparing the SBB contrast in keratinocytes previously treated with blend 1 or blend 2, looking individually at each of the experiments, blend 1 was found to exhibit a smaller variation in lipofuscin build up as compared to its non-irradiated control, suggesting greater influence on protecting against build up of this pigment (Figure 30).

### Partial conclusion of anti-aging effect

The anti-aging effect can be explained by the antioxidant and antiradical activities as well as protection of membranes from the actives, which prevent the accumulation of oxidative damage, preserving biomolecules and organelles and reducing lipofuscin build up.

The biochemical and cellular DPPH results demonstrate the greater capacity of the antioxidant cosmetic complex according to the present invention to neutralize free radicals as compared to the ingredients alone.

Furthermore, results of excited state suppression, membrane protection and anti-aging experiments show that the antioxidant cosmetic complex according to the present invention has the ability to protect membranes, as well as to increase cell viability and reduce cell aging.

The antioxidant cosmetic complex according to the present invention has shown greater capacity to protect from the accumulation of oxidative damage, preserving biomolecules and organelles and inhibiting the lipofuscin build up to a greater extent as compared to its irradiated control.

Thus, considering all the results presented together, the antioxidant cosmetic complex according to the present invention was shown to be surprisingly more effective in neutralizing free radicals and inhibiting the accumulation of oxidative damage.

With regard to the antioxidant effect, the test results presented herein show a 20% increase in antioxidant protection, a 36% increase in the synthesis of endogenous antioxidants and a 60% increase in the protection from oxidative damage to the membrane with an antioxidant cosmetic complex according to the present invention.

Therefore, the complex according to the present invention can be used in emulsions, nanoemulsions, suspensions, sunscreens, powders, waxes, oils, or any other cosmetic form applicable in cosmetics.

The person skilled in the art will be able to readily assess through the teachings of the instant text and examples the advantages of the invention and to propose variations and equivalent alternatives of implementation without departing from the scope of the invention as defined in the appended claims.

## Claims

1. An ANTIOXIDANT COSMETIC COMPLEX **characterized in that** it comprises *Euterpe oleracea, Theobroma cacao* and *Inga edulis.*

2. The COMPLEX according to claim 1, **characterized in that** it comprises:
(a) from about 0.0002% to about 20% of *Euterpe oleracea;*
(b) from about 0.0001% to about 20% of *Theobroma cacao;*
(c) from about 0.0002% to about 20% of *Inga edulis;*
based on the total weight of the cosmetic composition.

3. A COSMETIC COMPOSITION **characterized in that** it comprises the antioxidant cosmetic complex as defined in any of claims 1 or 2 together with cosmetically acceptable excipients.

4. USE of the complex as defined in any of claims 1 or 2, **characterized in that** it is in the manufacture of a cosmetic composition to act against damage caused by oxidative stress and free radicals in the skin and/or scalp.

5. A METHOD FOR COSMETIC TREATMENT **characterized in that** it consists of applying to the skin and/or scalp a complex as defined in any of claims 1 or 2.
